(19) Europäisches Patentamt
European Patent Office
Office européen des brevets

(11) **EP 4 223 886 B1**

(12) **EUROPEAN PATENT SPECIFICATION**

(45) Date of publication and mention of the grant of the patent:
**02.10.2024 Bulletin 2024/40**

(21) Application number: **23164521.9**

(22) Date of filing: **16.04.2020**

(51) International Patent Classification (IPC):
**C12Q 1/6806** (2018.01)

(52) Cooperative Patent Classification (CPC):
(C-Sets available)
**C12Q 1/6876; C12Q 1/6806;** C12Q 2600/166;
C12Q 2600/178 (Cont.)

(54) **COMPOSITIONS AND METHODS FOR THE STABILIZATION OF MICRO-RNA**

ZUSAMMENSETZUNGEN UND VERFAHREN ZUR STABILISIERUNG VON MIKRO-RNA

COMPOSITIONS ET PROCÉDÉS POUR LA STABILISATION DE MICRO-ARN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA TN**

(30) Priority: **16.04.2019 EP 19305496**

(43) Date of publication of application:
**09.08.2023 Bulletin 2023/32**

(62) Document number(s) of the earlier application(s) in accordance with Art. 76 EPC:
**20717908.6 / 3 956 470**

(73) Proprietor: **Genfit**
**59120 Loos (FR)**

(72) Inventor: **MAJD, Zouher**
**59320 Ennetieres-en-Weppes (FR)**

(74) Representative: **Cabinet Becker et Associés**
**25, rue Louis le Grand**
**75002 Paris (FR)**

(56) References cited:
WO-A1-2013/148151

• **GUO ZE ET AL: "MiR-23a regulates DNA damage repair and apoptosis in UVB-irradiated HaCaT cells", JOURNAL OF DERMATOLOGICAL SCIENCE, vol. 69, no. 1, 1 January 2013 (2013-01-01), pages 68 - 76, XP028962041, ISSN: 0923-1811, DOI: 10.1016/J.JDERMSCI.2012.10.014**
• **SMITH SIOBHÁN ET AL: "MicroRNA-302d targets IRF9 to regulate the IFN-induced gene expression in SLE", JOURNAL OF AUTOIMMUNITY, LONDON, GB, vol. 79, 17 March 2017 (2017-03-17), pages 105 - 111, XP029969306, ISSN: 0896-8411, DOI: 10.1016/J.JAUT.2017.03.003**

Remarks:
The complete document including Reference Table(s) and the Sequence Listing(s) can be downloaded from the EPO website

(52) Cooperative Patent Classification (CPC): (Cont.)

C-Sets
**C12Q 1/6806, C12Q 2523/31, C12Q 2525/207,
C12Q 2527/125, C12Q 2527/143, C12Q 2545/101,
C12Q 2545/107**

**Description**

The present invention relates to the stabilization of micro-RNA (miRNA) molecules. The compositions and methods described herein can advantageously be used for the provision of standard microRNAs for inclusion into kits, useful for the relative or absolute quantification of a miRNA in a biological fluid.

Circulating miRNAs offer many features to make them an attractive class of biomarkers. They are stable, their sequences are evolutionarily conserved, miRNA expression is often tissue or pathology specific, and because they can be detected by real-time PCR, assays can be highly sensitive and specific. However, there are also challenges associated with the detection of circulating miRNAs that still need to be addressed. One of the challenges relates to the low amount of total RNA in blood, which makes it difficult to measure the concentration and quality of the isolated RNA. As a consequence, it is of crucial importance to i) have calibrated and precise positive controls of miRNAs of interest and ii) precisely normalize detected miRNA values for variances based on the amount of starting material and miRNA extraction. Normalization has been tried by seeking a "housekeeping" circulating RNA. Some reports use U6 or other miRNAs (e.g., miR-16) as a housekeeping RNA. However, the levels of these RNAs often change under pathological conditions. Others have reported a spiked-in normalization approach in which 3 synthetic *Caenorhabditis elegans* miRNAs (without homology to human miRNAs) were added during the purification procedure and used for data normalization. However, while miRNAs are relatively stable in biological fluids, being associated to proteins and lipids, and packaged into macrovesicles, synthetic miRNAs are easily hydrolyzed by RNases and are thus difficult to handle or use. This is a major concern because a spiked-in approach should use a miRNA standard of known concentration, a requirement for absolute or relative quantification of a miRNA level from a biological fluid.

WO 2013/148151 describes that synthesized miRNAs are mixed with LipoFectamine before spiked in plasma to be protected from degradation. Guo ze et al. (Journal of Dermatological Science 2013 vol 69 p68-76) describe the use of lipofectamine as a lipofecting agent to transfect cells with miRNA.

The present invention provides compositions and methods solving this issue.

The inventors herein show that synthetic miRNAs can advantageously be introduced into kits useful for the diagnosis, prognosis or monitoring of a disease when complexed with a lipid vector.

Accordingly, a first aspect of the invention relates to a diagnostic kit comprising more than one container comprising a synthetic miRNA and a lipid vector, wherein said synthetic miRNA is at a defined concentration, wherein the lipid vector is selected from lipids conjugated to PEG.

In a particular aspect of the invention relates to a diagnostic kit comprising more than one container comprising a synthetic miRNA, a lipid vector and a matrix (for example synthetic serum, plasma depleted in nucleic acid, blood derived sample), wherein said synthetic miRNA is at a defined concentration.

The kit comprises:

- one or more container(s) comprising a first synthetic miRNA; and
- one or more container(s) comprising a second synthetic miRNA different from the first miRNA.

For example, the kit can comprise:

- a first set of containers containing a first synthetic miRNA, wherein said first synthetic miRNA is at a different concentration in each container of the first set of containers; and
- a second set of containers containing a second synthetic miRNA, wherein said second synthetic miRNA is at a different concentration in each container of the second set of containers.

The kit can further comprise at least one other container or at least one other set of containers comprising an additional synthetic miRNA (e.g. a third or more than third synthetic miRNA). The additional synthetic miRNA can be different from the first synthetic miRNA, from the second synthetic miRNA and from any other miRNA of a lower order included into the kit. For example, in case of a third synthetic miRNA, said third synthetic miRNA is different from the first synthetic miRNA and from the second synthetic miRNA. In the embodiment comprising another set of containers, the additional synthetic miRNA (e.g. the third synthetic miRNA) can be at a different concentration in each container of the additional (e.g. third) set of containers.

The synthetic miRNA(s) included in the kit of the invention can be any synthetic miRNA that can be used for quality control or for internal control, diagnostic, prognostic or monitoring value. For example, the synthetic miRNA(s) included in the kit can have the sequence of a miRNA whose level, absence or presence in a biological fluid is correlated to a disease or disease stage, or is potentially correlated to a disease or disease stage, or is correlated to a potential disease or potential disease stage. Illustrative diseases whose diagnosis can comprise the assessment of the level, presence or absence of at least one miRNA include, without limitation, non-alcoholic fatty liver disease (NAFLD), NASH, cancers, fibrosis, viral infections, nervous system disorders, cardiovascular disorders and diabetes.

In a particular embodiment, the synthetic miRNA(s) is a miRNA whose level, absence or presence is associated to NAFLD, NASH or fibrosis, in particular to NASH with fibrosis.

In a particular embodiment, at least one of the more than one container(s) comprises synthetic hsa-miR-34a (more particularly hsa-miR-34a-5p), hsa-miR-452 (more particularly hsa-miR-452-5p) or synthetic hsa-miR-193 (more particularly hsa-miR-193b-3p).

In a further particular embodiment, at least one of the more than one container(s) comprises synthetic hsa-miR-34a (more particularly hsa-miR-34a-5p). In yet another embodiment, the kit comprises a set of containers each comprising synthetic hsa-miR-34a (more particularly hsa-miR-34a-5p) at a different concentration. In a particular variant, said kit comprises one, at least two or at least three containers each comprising synthetic hsa-miR-34a (more particularly hsa-miR-34a-5p) at a different concentration. In another particular variant, said kit comprises one, two or three containers each comprising synthetic hsa-miR-34a (more particularly hsa-miR-34a-5p) at a different concentration. A further variant relates to a kit comprising one container comprising synthetic hsa-miR-34a (more particularly hsa-miR-34a-5p) at a defined concentration. Another variant relates to a kit comprising two containers comprising synthetic hsa-miR-34a (more particularly hsa-miR-34a-5p) at a defined concentration, wherein the concentration of synthetic hsa-miR-34a (more particularly hsa-miR-34a-5p) in the first container is different from the concentration of synthetic hsa-miR-34a (more particularly hsa-miR-34a-5p) in the second container. In yet a further variant, the kit comprises three containers comprising synthetic hsa-miR-34a (more particularly hsa-miR-34a-5p) at a defined concentration, wherein the concentration of synthetic hsa-miR-34a (more particularly hsa-miR-34a-5p) in the first container is different from the concentration of synthetic hsa-miR-34a (more particularly hsa-miR-34a-5p) in the second container, and wherein the concentration of synthetic hsa-miR-34a (more particularly hsa-miR-34a-5p) in the third container is different from the concentration of synthetic hsa-miR-34a (more particularly hsa-miR-34a-5p) in the first container and in the second container.

In a further particular embodiment, at least one of the more than one container(s) comprises synthetic hsa-miR-193 (more particularly hsa-miR-193b-3p). In yet another embodiment, the kit comprises a set of containers each comprising synthetic hsa-miR-193 (more particularly hsa-miR-193b-3p) at a different concentration. In a particular variant, said kit comprises one, at least two or at least three containers each comprising synthetic hsa-miR-193 (more particularly hsa-miR-193b-3p) at a different concentration. In another particular variant, said kit comprises one, two or three containers each comprising synthetic hsa-miR-193 (more particularly hsa-miR-193b-3p) at a different concentration. A further variant relates to a kit comprising one container comprising synthetic hsa-miR-193 (more particularly hsa-miR-193b-3p) at a defined concentration. Another variant relates to a kit comprising two containers comprising synthetic hsa-miR-193 (more particularly hsa-miR-193b-3p) at a defined concentration, wherein the concentration of synthetic hsa-miR-193 (more particularly hsa-miR-193b-3p) in the first container is different from the concentration of synthetic hsa-miR-193 (more particularly hsa-miR-193b-3p) in the second container. In yet a further variant, the kit comprises three containers comprising synthetic hsa-miR-193 (more particularly hsa-miR-193b-3p) at a defined concentration, wherein the concentration of synthetic hsa-miR-193 (more particularly hsa-miR-193b-3p) in the first container is different from the concentration of synthetic hsa-miR-193 (more particularly hsa-miR-193b-3p) in the second container, and wherein the concentration of synthetic hsa-miR-193 (more particularly hsa-miR-193b-3p) in the third container is different from the concentration of synthetic hsa-miR-193 (more particularly hsa-miR-193b-3p) in the first container and in the second container.

In a particular embodiment, at least one of the more than one container(s) comprises synthetic cel-miR-39 (more particularly cel-miR-39-3p). In yet another embodiment, the kit comprises a set of containers each comprising synthetic cel-miR-39 (more particularly cel-miR-39-3p) at a different concentration. In a particular variant, said kit comprises one, at least two or at least three containers each comprising synthetic cel-miR-39 (more particularly cel-miR-39-3p) at a different concentration. In another particular variant, said kit comprises one, two or three containers each comprising synthetic cel-miR-39 (more particularly cel-miR-39-3p) at a different concentration. A further variant relates to a kit comprising one container comprising synthetic cel-miR-39 (more particularly cel-miR-39-3p) at a defined concentration. Another variant relates to a kit comprising two containers comprising synthetic cel-miR-39 (more particularly cel-miR-39-3p) at a defined concentration, wherein the concentration of synthetic cel-miR-39 (more particularly cel-miR-39-3p) in the first container is different from the concentration of synthetic cel-miR-39 (more particularly cel-miR-39-3p) in the second container. In yet a further variant, the kit comprises three containers comprising synthetic cel-miR-39 (more particularly cel-miR-39-3p) at a defined concentration, wherein the concentration of synthetic cel-miR-39 (more particularly cel-miR-39-3p) in the first container is different from the concentration of synthetic cel-miR-39 (more particularly cel-miR-39-3p) in the second container, and wherein the concentration of synthetic cel-miR-39 (more particularly cel-miR-39-3p) in the third container is different from the concentration of synthetic cel-miR-39 (more particularly cel-miR-39-3p) in the first container and in the second container.

In another particular embodiment, at least one of the more than one container(s) comprises synthetic cel-miR-40 (more particularly cel-miR-40-3p). In yet another embodiment, the kit comprises a set of containers each comprising synthetic cel-miR-40 (more particularly cel-miR-40-3p) at a different concentration. In a particular variant, said kit comprises one, at least two or at least three containers each comprising synthetic cel-miR-40 (more particularly cel-miR-40-3p) at a different concentration. In another particular variant, said kit comprises one, two or three containers each comprising synthetic cel-miR-40 (more particularly cel-miR-40-3p) at a different concentration. A further variant relates to a kit comprising one container comprising synthetic cel-miR-40 (more particularly cel-miR-40-3p) at a defined concentration. Another variant relates to a kit comprising two containers comprising synthetic cel-miR-40 (more particularly cel-miR-40-3p) at a defined concentration, wherein the concentration of synthetic cel-miR-40 (more particularly cel-miR-40-3p) in the first container is different from the concentration of synthetic cel-miR-40 (more particularly cel-miR-40-3p) in the second container. In yet a further variant, the kit comprises three containers comprising synthetic cel-miR-40 (more particularly cel-miR-40-3p) at a defined concentration, wherein the concentration of synthetic cel-miR-40 (more particularly cel-miR-40-3p) in the first container is different from the concentration of synthetic cel-miR-40 (more particularly cel-miR-40-3p) in the second container, and wherein the concentration of synthetic cel-miR-40 (more particularly cel-miR-40-3p) in the third container is different from the concentration of synthetic cel-miR-40 (more particularly cel-miR-40-3p) in the first container and in the second container.

In a further particular embodiment, at least one of the more than one container(s) comprises synthetic ath-miR-159a, cel-lin-4, cel-miR-2, cel-miR-238, cel-miR-54 or cel-miR-55, more particularly ath-miR-1549a, cel-lin4-5p, cel-miR2-3p, cel-miR-238-3p, cel-miR-54-3p or cel-miR-55-3p respectively.

The container(s) of the kit of the disclosure comprises a complex of a miRNA and of a lipid vector. Illustrative lipid vectors can comprise, without limitation, cationic lipids, non-cationic lipids (such as neutral or anionic lipids) and conjugated lipids, such as lipids conjugated to amino-acids, lipids conjugated to peptides, or lipids conjugated to PEG. In an embodiment, lipid vectors for practice of the invention comprise non-cationic lipids (such as neutral or anionic lipids) and lipids conjugated to PEG. In a particular embodiment, the lipid vector comprises at least one cationic lipid. In a further particular embodiment, the at least one cationic lipid is mixed with at least one neutral lipid and/ or to at least one conjugated lipid. In a further particular embodiment, a cationic lipid can be mixed with PEG, such as C12-C20, in particular C14-C18, PEG with 500 - 7500 molecular weight.

The lipid vector for practice of the invention is selected from lipids conjugated to PEG. In a particular embodiment, the lipid vector is a glycerophospholipid conjugated to PEG. In yet another embodiment, the lipid vector is a monoglycerophosphoethanolamine conjugated to PEG or a diacylglycerophosphoethanolamine conjugated to PEG, or a salt thereof. In a further embodiment, the lipid vector is a diacylglycerophosphoethanolamine conjugated to PEG, or a salt thereof. In another particular embodiment, the lipid vector is a di($C_{14}$-$C_{18}$)acylglycerophosphoethanolamine conjugated to PEG, or a salt thereof, such as a di$C_{16}$acylglycerophosphoethanolamine conjugated to PEG, or a salt thereof. In each of the embodiments disclosed in the present paragraph, the PEG moiety may be a PEG having a molecular weight from 500 to 7500, in particular from 750 to 5000, such as from 1000 to 4000. In particular embodiment, the PEG moiety of the lipid vectors described in the present paragraph may be of 1000, 2000, 3000 or 4000, in particular 2000. In a further particular embodiment, the lipid vector is 1,2-dipalmitoyl-sn-glycero-3-phosphoethanolamine-N-[methoxy(polyethylene glycol)-2000] (C16 PEG2000 PE), or a salt thereof such as its ammonium salt.

In a particular embodiment, the cationic lipid is a monovalent or polyvalent cationic lipid. Among monovalent cationic lipids, one can cite, without limitation, DOTMA, DOTAP, DMRIE, and DDAB. Illustrative polyvalent cationic lipids include, without limitation, DOSPA, DOSPER, DOGS, TMTPS, TMTOS, TMTLS, TMTMS, and TMDOS. Among neutral lipids, one can cite, without limitation, DOPE, DPhPE, and cholesterol.

For example, illustrative lipid vectors include, without limitation, those disclosed in WO9405624, WO9427435, WO9502698, WO9517373, WO9640961, US8058068, WO9840502, US20060229246, US20030069173, WO200027795, WO200234879, WO2006102163, WO201268176, WO201611203 and US20190060482.

In a particular embodiment of the disclosure, the lipid vector is selected from:

2,3-dioleyloxy-1 -(N,N-dimethylamino)propane;
2H-Isoindole-2-ethanaminium, N-[2,3-bis(9-octadecenyloxy)propyl]-1,3-dihydro-N,N-dimethyl-1,3-dioxo-, bromide;
1-Propanaminium. N- (2 -amino) ethyl-N, N-dimethyl- 2,3 -bis (9-octadecenyloxy) - bromide; 3-Oxa-5,9,15-triazaheptadecan-17-aminium, N-[2,3-bis(9-octadecenyloxy)propyl]-9-[(1,1-dimethylethoxy)carbonyl]-13-{[(1,1-dimethylethoxy)carbonyl] [3-{[(1,1-dimethylethomy)-carbonyl]aminolpropyl]aminol-N,N,2,2-tetramethyl-4,14-dioxo-, bromide;
1-Propanaminium. N-[2-[[2,5-bis[(3-aminopropyl)amino]-1-oxo-pentyl]amino]ethyl]-n,n-dimethyl-2,3,-bis(9-octadecenyloxy)-, tetra(trifluoroacetate) salt
1-Propanaminium, N-[2-(2-bromo)ethyl]-N,N-dimethyl-2,3-bis(9-octadecenyloxy)-, bromide; 1-Propanaminium,N-{2-[[3-[[4-[(3-aminopropyl)amino]butyl]amino]propyl]amino]ethyl}-n,n-dimethyl-2,3-bis-(9-octadecenyloxy)-, bromide; and
1-Propanaminium, N-[2-[(3-aminopropyl)amino]ethyl]-N,N-dimethyl-2,3-bis(9-octadecenyloxy)-, bromide.

In a particular embodiment of the disclosure, the lipid vector is selected from:

N,N,N',N'-tetrapalmitoylspermine mixed or not with DOPE;

N,N,N',N'-tetrapalmylspermine mixed or not with DOPE;
N,N,N',N'-tetramethyltetrapalmylsperminetetrammonium iodide mixed or not with DOPE;
N,N,N',N'-hexamethyltetrapalmylsperminetetrammonium iodide;
DOTMA:DOPE (in particular 1:1 (w/w))and
DOSPA:DOPE (in particular 1.5:1 (w/w));

In a particular embodiment of the disclosure, the lipid vector is N,N,N',N'-tetramethyltetrapalmylsperminetetrammonium iodide, more particularly a mixture 1:1 mixture of N,N,N',N'-tetramethyltetrapalmylsperminetetrammonium iodide and DOPE;

In a particular embodiment, the lipid-based vector comprises 2,3-dioleyloxy-N-[2(sperminecarboxamido)ethyl]-N,N-dimethyl-1-propanaminium trifluoroacetate. In a further embodiment, 2,3-dioleyloxy-N-[2(sperminecarboxamido)ethyl]-N,N-dimethyl-1-propanaminium trifluoroacetate is mixed to a neutral lipid such as dioleoylphosphatidylethanolamine.

In another particular embodiment, the lipid-based vector comprises:

$N^1,N^4$-dioleoyl-diaminobutane
$N^1,N^4$-dioleyl-diaminobutane
$N^1,N^4$-dioleyl- $N^1,N^4$-di-[2-hydroxy-3-(N-phthalamido)propyl]-diamino-butane
$N^1,N^4$-dioleyl- $N^1,N^4$-di-[2-hydroxy-3-(N-aminopropyl)]- diaminobutane (DHDOS)
$N^1,N^4$-dimyristyl- $N^1,N^4$-di-[2-hydroxy-3-(N-aminopropyl)-diaminobutane;
$N^1,N^4$-dipalmityl- $N^1,N^4$-di-[2-hydroxy-3-(N-aminopropyl)-diamino butane;
$N^1,N^4$-dipalmitolyl- $N^1,N^4$-di-[2-hydroxy-3-(N-aminopropyl)-diaminobutane;
$N^1,N^4$-distearyl- $N^1,N^4$-di-[2-hydroxy-3-(N-aminopropyl)-diaminobutane;
$N^1,N^4$-dilauryl- $N^1,N^4$-di-[2-hydroxy-3-(N-aminopropyl)-diaminobutane;
$N^1,N^2$-dimyristyl- $N^1,N^2$-di-[2-hydroxy-3-(N-aminopropyl)-diaminoethane;
$N^1,N^2$-dipalmityl- $N^1,N^2$-di-[2-hydroxy-3-(N-aminopropyl)-diaminoethane;
$N^1,N^2$-dipalmitolyl- $N^1,N^2$-di-[2-hydroxy-3-(N-aminopropyl)-diaminoethane;
$N^1,N^2$-distearyl- $N^1,N^2$-di-[2-hydroxy-3-(N-aminopropyl)-diaminoethane;
$N^1,N^2$-dilauryl- $N^1,N^2$-di-[2-hydroxy-3-(N-aminopropyl)-diaminoethane;
$N^1,N^2$-dioleyl- $N^1,N^2$-di-[2-hydroxy-3-(N-aminopropyl)-diaminoethane;
$N^1,N^9$-dimyristyl- $N^1,N^9$-di-[2-hydroxy-3-(N-aminopropyl)-Jeffamine;
$N^1,N^9$-dipalmityl- $N^1,N^9$-di-[2-hydroxy-3-(N-aminopropyl)-Jeffamine;
$N^1,N^9$-dipalmitolyl- $N^1,N^9$-di-[2-hydroxy-3-(N-aminopropyl)-Jeffamine;
$N^1,N^9$-distearyl- $N^1,N^9$-di-[2-hydroxy-3-(N-aminopropyl)-Jeffamine;
$N^1,N^9$-dilauryl- $N^1,N^9$-di-[2-hydroxy-3-(N-aminopropyl)-Jeffamine;
$N^1,N^9$-dioleyl- $N^1,N^9$-di-[2-hydroxy-3-(N-aminopropyl)-Jeffamine
$N^1,N^4$-dioleyl-$N^1,N^4$-di-[2-hydroxy-3-(N-carboxamidine)aminopropyl]-diaminobutane;
$N^1,N^4$-dioleyl-$N^1,N^4$-di-{2-hydroxy-3-[N-(NI,NII,NIII,NIV-butoxycarbonyl-spermine carboxamido) aminopropyl) diaminobutane;
$N^1,N^4$-dioleyl-$N^1,N^4$-di-[2-hydroxy-3-(N-sperminecarboxamido)-aminopropyl]-diaminobutane.
$N^1,N^4$-dimyristyl- $N^1,N^4$-di-[2-hydroxy-3-(N-sperminecarboxamido )aminopropyl]-di amino butane;
$N^1,N^4$-dipalmityl- $N^1,N^4$-di-[2-hydroxy-3-(N-sperminecarboxamido )-aminopropyl ]-diaminobutane;
$N^1,N^4$-dipalmitolyl- $N^1,N^4$-di-[2-hydroxy-3-(N-sperminecarboxamido )aminopropyl]-diaminobutane;
$N^1,N^4$-distearyl- $N^1,N^4$-di-[2-hydroxy-3-(N-sperminecarboxamido)-aminopropyl]-diaminobutane;
$N^1,N^4$-dilauryl- $N^1,N^4$-di-[2-hydroxy-3-(N-sperminecarboxamido)-aminopropyl]diaminobutane;
N1 ,N9-dimyristyl-N1 ,N9-di-[2-hydroxy-3-(N-sperminecarboxamido )aminopropyl]-Jeffamine;
$N^1,N^9$ -dipalmityl- $N^1,N^9$-di-[2-hydroxy-3-(N-sperminecarboxamido )-aminopropyl ]-Jeffamine;
$N^1,N^9$-dipalmitolyl- $N^1,N^9$-di-[2-hydroxy-3-(N-sperminecarboxamido )aminopropyl]-Jeffamine;
$N^1,N^9$-distearyl- $N^1,N^9$-di-[2-hydroxy-3-(N-sperminecarboxamido)-aminopropyl]-Jeffamine;
$N^1,N^9$-dilauryl- $N^1,N^9$-di-[2-hydroxy-3-(N-sperminecarboxamido )-aminopropyl]Jeffamine;
$N^1,N^9$-dioleyl- $N^1,N^9$-di-[2-hydroxy-3-(N-sperminecarboxamido )-aminopropyl]Jeffamine;
$N^1,N^2$-dimyristyl- $N^1,N^2$-di-[2-hydroxy-3-(N-sperminecarboxamido )-aminopropyl ]-diaminoethane;
$N^1,N^2$-dipalmityl- $N^1,N^2$-di-[2-hydroxy-3-(N-sperminecarboxamido )aminopropyl]-diaminoethane;
$N^1,N^2$-dipalmitolyl- $N^1,N^2$-di-[2-hydroxy-3-(N-sperminecarboxamido )-aminopropyl]-diaminoethane;
$N^1,N^2$-distearyl- $N^1,N^2$-di-[2-hydroxy-3-(N-sperminecarboxamido)-aminopropyl]diaminoethane;
$N^1,N^2$-dilauryl- $N^1,N^2$-di-[2-hydroxy-3-(N-sperminecarboxamido)-aminopropyl]diaminoethane;
$N^1,N^2$-dioleyl- $N^1,N^2$-di-[2-hydroxy-3-(N-sperminecarboxamido )-aminopropyl ]-diaminoethane.

In a further embodiment, the lipid-based vector comprises:

N,N',N'',N''' tetramethyltetrapalmitylspermine (TMTPS-iodide or TMTPS-chloride);
N,N',N'',N''' tetramethyltetrapalmitylspermine (TMTPS-iodide) mixed to DOPE ((e.g. 1:1.5 (M/M)); a liposomal N,N',N'',N''' tetramethyltetrapalmitylspermine (TMTPS-iodide) mixed to DOPE (e.g. 1:1.5 (M/M)).

In another embodiment, the lipid-based vector comprises an amine-containing lipid such as:

Tetradecyl 2-[3-Methylaminopropyl-(2-oxo-2-tetradecoxyethyl)amino] Acetate;
2-[2-[2-[2-[bis [2-(Dodecylamino )-2-oxo-ethyl] amino] ethyl-[2-( dodecylamino )-2-oxo-ethyl] amino] ethylamino] ethyl-[2-( dodecylamino )-2-oxo-ethyl] amino ]-N-dodecyl-acetamide;
2-[2-[2-[2-[bis[2-(Dodecylamino)-2-oxoethyl]amino] ethylamino] ethylamino] ethyl-[2-( dodecylamino )-2-oxoethyl]amino ]-N-dodecyl-acetamide;
Dipentadecyl 4,4'-(3-(methyl(4-oxo-4-(pentadecyloxy)butyl)amino)propylazanediyl)Dibutanoate; and Pentadecyl 4-(methyl(3-( 4-oxo-4-(pentadecyloxy) butylamino )propyl)amino )Butanoate.

In a further particular embodiment, the lipid vector comprises at least one of compounds 1 to 87 of WO2012068176. In a further particular embodiment, the lipid vector comprises at least one lipid selected in the group consisting of:

Compound 1 of WO2012068176;
Compound 2 of WO2012068176;
Compound 3 of WO2012068176;
Compound 4 of WO2012068176;
Compound 5 of WO2012068176;
Compound 6 of WO2012068176;
Compound 7 of WO2012068176;
Compound 8 of WO2012068176;
Compound 9 of WO2012068176;
Compound 10 of WO2012068176;
Compound 11 of WO2012068176;
Compound 12 of WO2012068176;
Compound 13 of WO2012068176;
Compound 14 of WO2012068176;
Compound 15 of WO2012068176;
Compound 16 of WO2012068176;
Compound 17 of WO2012068176;
Compound 18 of WO2012068176;
Compound 19 of WO2012068176;
Compound 20 of WO2012068176;
Compound 21 of WO2012068176;
Compound 22 of WO2012068176;
Compound 23 of WO2012068176;
Compound 24 of WO2012068176;
Compound 25 of WO2012068176;
Compound 26 of WO2012068176;
Compound 27 of WO2012068176;
Compound 28 of WO2012068176;
Compound 29 of WO2012068176;
Compound 30 of WO2012068176;
Compound 31 of WO2012068176;
Compound 32 of WO2012068176;
Compound 33 of WO2012068176;
Compound 34 of WO2012068176;
Compound 35 of WO2012068176;
Compound 36 of WO2012068176;
Compound 37 of WO2012068176;
Compound 38 of WO2012068176;

Compound 39 of WO2012068176;
Compound 40 of WO2012068176;
Compound 41 of WO2012068176;
Compound 42 of WO2012068176;
Compound 43 of WO2012068176;
Compound 44 of WO2012068176;
Compound 45 of WO2012068176;
Compound 46 of WO2012068176;
Compound 47 of WO2012068176;
Compound 48 of WO2012068176;
Compound 49 of WO2012068176;
Compound 50 of WO2012068176;
Compound 51 of WO2012068176;
Compound 52 of WO2012068176;
Compound 53 of WO2012068176;
Compound 54 of WO2012068176;
Compound 55 of WO2012068176;
Compound 56 of WO2012068176;
Compound 57 of WO2012068176;
Compound 58 of WO2012068176;
Compound 59 of WO2012068176;
Compound 60 of WO2012068176;
Compound 61 of WO2012068176;
Compound 62 of WO2012068176;
Compound 63 of WO2012068176;
Compound 64 of WO2012068176;
Compound 65 of WO2012068176;
Compound 66 of WO2012068176;
Compound 67 of WO2012068176;
Compound 68 of WO2012068176;
Compound 69 of WO2012068176;
Compound 70 of WO2012068176;
Compound 71 of WO2012068176;
Compound 72 of WO2012068176;
Compound 73 of WO2012068176;
Compound 74 of WO2012068176;
Compound 75 of WO2012068176;
Compound 76 of WO2012068176;
Compound 77 of WO2012068176;
Compound 78 of WO2012068176;
Compound 79 of WO2012068176;
Compound 80 of WO2012068176;
Compound 81 of WO2012068176;
Compound 82 of WO2012068176;
Compound 83 of WO2012068176;
Compound 84 of WO2012068176;
Compound 85 of WO2012068176;
Compound 86 of WO2012068176;
Compound 87 of WO2012068176; and
salts thereof.

In another specific embodiment, the lipid vector can be a lipid formulation as disclosed in tables 1, 2 and 3 of WO2012068176, which are reproduced below:

Table 1 of WO2012068176

| Compound of WO2012068176 | PEG Lipid | Helper Lipid | Buffer | Ethanol, % | Concentration, mg/ml |
|---|---|---|---|---|---|
| 49 | C16 PEG2000 PE | Cholesterol | 50mM Sodium Acetate | 25 | 15.625 |
| 7 | C16 PEG2000 PE | Cholesterol | 50mM Sodium Acetate | 25 | 15.625 |
| 69 | C14 PEG2000 PE | Cholesterol | 50mM Sodium Acetate | 25 | 15.625 |
| 70 | C14 PEG2000 PE | Cholesterol | 50mM Sodium Acetate | 25 | 15.625 |
| 51 | C14 PEG2000 PE | Cholesterol | 50mM Sodium Acetate | 25 | 15.625 |
| 52 | C14 PEG2000 PE | Cholesterol | 50mM Sodium Acetate | 25 | 15.625 |
| 54 | C14 PEG2000 PE | Cholesterol | 50mM Sodium Acetate | 25 | 15.625 |
| 56 | C16 PEG2000 PE | Cholesterol | 50mM Sodium Acetate | 25 | 15.625 |
| 57 | C16 PEG2000 PE | Cholesterol | 50mM Sodium Acetate | 25 | 15.625 |
| 73 | C16 PEG2000 PE | Cholesterol | 50mM Sodium Acetate | 25 | 15.625 |
| 76 | C16 PEG2000 PE | Cholesterol | 50mM Sodium Acetate | 25 | 15.625 |
| 77 | C16 PEG2000 PE | Cholesterol | 50mM Sodium Acetate | 25 | 15.625 |

Table 2 of WO2012068176:

| Compound of WO2012068176 | PEG Lipid | Helper Lipid | Buffer | Ethanol, % | Concentration, mg/ml |
|---|---|---|---|---|---|
| IVF2.0 | C16 PEG2000 PE | Cholesterol | 139.5mM Sodium Acetate | 25 | 15.625 |
| 57 NO (CB00396) | C16 PEG2000 PE | Cholesterol | 139.5mM Sodium Acetate | 25 | 15.625 |

(continued)

| Compound of WO2012068176 | PEG Lipid | Helper Lipid | Buffer | Ethanol, % | Concentration, mg/ml |
|---|---|---|---|---|---|
| 57 OPT (CB00396) | C14 PEG2000 PE | Cholesterol | 139.5mM Sodium Acetate | 25 | 15.625 |
| 72 OPT (CB00401) | C14 PEG2000 PE | Cholesterol | 139.5mM Sodium Acetate | 25 | 15.625 |
| 84 NO (CB00416) | C14 PEG2000 PE | Cholesterol | 139.5mM Sodium Acetate | 25 | 15.625 |
| 84 OPT (CB00416) | C14 PEG2000 PE | Cholesterol | 139.5mM Sodium Acetate | 25 | 15.625 |

| Compound of WO2012068176 | Helper Lipid | | Ethanol, % |
|---|---|---|---|
| 83 | None | | 100 |
| 67 | Cholesterol | | 100 |

In a further particular embodiment of the disclosure, the lipid vector is a mixture of DHDMS, HDMS, DOPE and cholesterol. In another embodiment, the lipid vector is a lipid formulation as disclosed in table 1 of WO2016011203:

Table 1 of WO2016011203:

| Formulation | DHDMS | HDMS | DOPE | Cholesterol |
|---|---|---|---|---|
| 1 | 0.1 | 0.1 | 0.4 | 0.4 |
| 2 | 0.1 | 0.25 | 0.25 | 0.4 |
| 3 | 0.1 | 0.25 | 0.4 | 0.25 |
| 4 | 0.1 | 0.4 | 0.1 | 0.4 |
| 5 | 0.1 | 0.4 | 0.25 | 0.25 |
| 6 | 0.1 | 0.4 | 0.4 | 0.1 |
| 7 | 0.1 | 0.3 | 0.3 | 0.3 |
| 8 | 0.2 | 0.4 | 0.2 | 0.2 |
| 9 | 0.2 | 0.2 | 0.2 | 0.4 |
| 10 | 0.2 | 0.2 | 0.4 | 0.2 |
| 11 | 0.25 | 0.4 | 0.1 | 0.25 |
| 12 | 0.25 | 0.4 | 0.25 | 0.1 |
| 13 | 0.25 | 0.1 | 0.25 | 0.4 |
| 14 | 0.25 | 0.1 | 0.4 | 0.25 |
| 15 | 0.25 | 0.25 | 0.1 | 0.4 |
| 16 | 0.25 | 0.25 | 0.4 | 0.1 |
| 17 | 0.3 | 0.1 | 0.3 | 0.3 |
| 18 | 0.3 | 0.3 | 0.1 | 0.3 |
| 19 | 0.3 | 0.3 | 0.3 | 0.1 |
| 20 | 0.4 | 0.4 | 0.1 | 0.1 |

(continued)

| Formulation | DHDMS | HDMS | DOPE | Cholesterol |
|---|---|---|---|---|
| 21 | 0.4 | 0.2 | 0.2 | 0.2 |
| 22 | 0.4 | 0.25 | 0.1 | 0.25 |
| 23 | 0.4 | 0.25 | 0.25 | 0.1 |
| 24 | 0.4 | 0.1 | 0.1 | 0.4 |
| 25 | 0.4 | 0.1 | 0.25 | 0.25 |
| 26 | 0.4 | 0.1 | 0.4 | 0.1 |

In a further particular embodiment, the lipid vector comprises a lipid nanoparticle formulation as disclosed in US20190060482, comprising PEG of 750, 2000 or 5000 molecular weight, with a chain length of 14 or 18, DHDMS, HDMS, DOPE, and cholesterol. More particularly, the formulation is one of the formulations of table 2 of US20190060482:

| Formulation | PEG chain length | PEG Mol Weight | DHDMS | HDMS | DOPE | Cholesterol | PEG |
|---|---|---|---|---|---|---|---|
| Bruce #3.10 | 14 | 5000 | 0.24 | 0.38 | 0.32 | 0.05 | 0.01 |
| Bruce #3.14 | 14 | 5000 | 0.32 | 0.39 | 0.26 | 0.01 | 0.02 |
| Bruce #3.20 | 14 | 5000 | 0.18 | 0.38 | 0.32 | 0.10 | 0.02 |
| Bruce #3.19 | 14 | 5000 | 0.23 | 0.45 | 0.20 | 0.10 | 0.02 |
| Bruce #3.11 | 14 | 5000 | 0.18 | 0.51 | 0.20 | 0.10 | 0.01 |
| Bruce #3.15 | 14 | 5000 | 0.27 | 0.38 | 0.32 | 0.01 | 0.02 |
| Bruce #3.12 | 14 | 5000 | 0.25 | 0.38 | 0.26 | 0.10 | 0.01 |
| Bruce #2.2 | 18 | 750 | 0.28 | 0.24 | 0.32 | 0.14 | 0.02 |
| Bruce #3.16 | 14 | 5000 | 0.18 | 0.47 | 0.32 | 0.01 | 0.02 |
| Bruce #4 | 14 | 2000 | 0.24 | 0.40 | 0.24 | 0.10 | 0.02 |
| Bruce #3.18 | 14 | 5000 | 0.32 | 0.38 | 0.20 | 0.08 | 0.02 |
| Molar ranges | | | 0.18-0.32 | 0.24-0.51 | 0.20-0.32 | 0.01-0.14 | 0.01-0.02 |

In a particular embodiment of the disclosure, the lipid vector is a lipid-based transfection reagent, such as a lipid-based transfection selected from:

Lipofectamine (3:1 (w/w) mixture of the polycationic lipid, 2,3-dioleyloxy-N-[2(sperminecarboxamido)ethyl]-N,N-dimethyl-1-propanaminium trifluoroacetate (DOSPA), and DOPE);
Lipofectamine 2000;
Lipofectace (1:2.5 (w/w) mixture of dimethyldioctadecylammonium bromide (DDAB) and dioleoylphosphatidylethanolamine (DOPE));
DORI-ether;
DORI-ether/lysolipid;
Lipofectin (1:1 (w/w) mixture of N-[1-(2,3-dioleyloxy)propyl]-N,N,N-trimethylammonium chloride (DOTMA) and dioleoylphosphatidylethanolamine (DOPE));
DOTAP;
BioPORTER;
Cellfectin (a 1:1.5 M/M liposome formulation of a cationic lipid tetramethylpalmitylspermine (TMTPS) and DOPE); and
DMRIE-C (a 1:1 M/M liposome formulation of a cationic lipid N-(2-hydroxyethyl)-N,N-dimethyl-2.3-bis(tetradecyloxy)-1-propanaminium bromide (DMRIE) and cholesterol).

In a particular embodiment of the invention, the lipid vector is invivofectamine® 2.0 or invivofectamine® 3.0, in particular invivofectamine® 3.0.
The invention also relates to uses of the diagnostic kit of the invention.

In a particular embodiment, the diagnostic kit of the invention is used in a method for the diagnosis of a disease. In particular, the kit is used to provide a calibration standard for the miRNA(s) contained in the kit.

In the context of the present invention, the expression "method for the diagnosis" denotes a method for the diagnosis, prognosis or monitoring of a disease, but also a method for evaluating the efficacy of a treatment against the disease.

The invention thus relates to the use of the diagnostic kit of the invention, in a method for the diagnosis, prognosis or monitoring of a disease. The kit of the invention may be a method for the diagnosis of non-alcoholic steatohepatitis (NASH), and for classifying a subject as a potential receiver of a treatment for NASH as described in the PCT application WO2017167934.

The invention further relates to a method for the diagnosis of a disease, comprising the determination of the level of miRNA in a biological sample of a subject, wherein the level of said miRNA is compared to the level of the same miRNA in the standard or positive control from the kit of the present invention.

In a particular embodiment, the uses and methods of the invention comprise the comparison of the level of the miRNA determined from the biological sample to the level of the synthetic miRNA used as a standard or positive control in the kit.

In another embodiment, the uses and methods of the invention comprise the normalization of the level of the miRNA determined from the biological sample to the level of the synthetic miRNA used as an internal control in the kit.

In a further embodiment, the uses and methods of the invention comprise the normalization of the level of the miRNA determined from the biological sample with the extraction yield of the synthetic miRNA used as a standard or positive control in the kit.

The uses and methods of the invention are useful for the quantification of miRNA in a biological fluid sample of a subject, such as blood derived samples, for example whole blood, serum or plasma, in particular serum.

In a further embodiment, the uses and methods of the invention comprise the spike in of a defined amount of the synthetic miRNA in the kit of the invention into the biological sample into which a miRNA level is to be determined. In a particular embodiment, the spike-in synthetic miRNA is a non-human miRNA, an exogenous miRNA absent in the subject sample. For example, the non-human synthetic or exogenous miRNA can be derived from *Caenorhabditis elegans (cel) or Arabidopsis thaliana* (*ath*). In a particular embodiment, the spiked-in miRNA is cel-miR39 (in particular cel-miR-39-3p). Other non-human miRNA for use as synthetic miRNA in the context of the present invention include, without limitation, ath-miR-159a and cel-miR-40-3p.

The kit of the invention can thus be used to provide an internal control measured simultaneously to the miRNA to be tested in the biological fluid sample. It can be used to calculate the level of miRNA in the sample using the following formula, which is given for an illustrative purpose with respect to the measurement of hsa-miR-34a-5p as the miRNA to be tested in the sample, and cel-miR-39-3p as the internal control and spiked synthetic miRNA:

$$\text{hsa-miR-34a-5p levels} = 2^{-\Delta\Delta Cq}$$

Where:

$$\Delta\Delta Cq = \Delta Cq_{\text{sample}} - \Delta Cq_{\text{Standard}}$$

$$\Delta Cq_{\text{sample}} = Cq_{\text{hsa-miR-34a-5p of the sample}} - Cq_{\text{cel-miR-39-3p spiked in the samples}}$$

$$\Delta Cq_{\text{standard}} = Cq_{\text{hsa-miR-34a-5p of the positive standard}} - Cq_{\text{cel-miR-39-3p spiked in the positive standard}}$$

The invention is defined in the appended claims.

EXAMPLES

### - Invivofectamine®/miRs complex preparation

Highly purified miRNAs oligoribonucleotides are custom synthesized from IDT (Integrated DNA Technologies Skokie, Illinois USA).

Invivofectamine® 3.0 reagent (IVF3001-3005, ThermoFisher Scientific, USA) was used for creating complexes with miRNAs according to manufacturer recommendations (Pub. no. MAN0013651 Rev A.0.pdf). The miRNA/Invivofectamine® (IVF) complex was used without dilution or diluted 6-fold by adding 1 mL PBS (pH 7.4).

*- Standards and internal controls preparation*

To prepare standards miRNAs or internal controls, 5 μL of preparation is added to 1) biological base matrices for standard preparations (i.e. serum or plasma from healthy donors, or other base matrix) or 2) directly to biological samples as internal control.

Standards miRNAs, positive controls and internal controls are prepared with synthetic miRNA oligoribonucleotide (i.e. hsa-miR-34a-5p, cel-miR-39-3p, cel-miR-40-3p).

*- Small RNA Extraction*

Samples to be tested are thawed on ice. Vortex gently the biological samples and centrifuge at 6,000xg for 15 minutes.

Automated extraction with MagMax mirVana™ on KingFisher™ System (KingFisher™ Flex System, catalog nbr 5400630, ThermoFisher) was carried out according to manufacturers recommendations (KingFisher™_Flex_User_Manual_5400630.pdf, part nbr N07669). MagMax mirVana™ Total RNA Isolation Kit (A27828, ThermoFisher) was used following the user guide (MagMAX mirVana™ Total RNA Isolation Kit (manual extraction) User Guide - Pub. no. MAN0011131 - Rev. B.0.pdf).

*- Reverse Transcription (RT)*

Reverse transcription reaction was carried using TaqMan® MicroRNA Reverse Transcription Kit, catalog nbr 4366597 following user manual protocol: TaqMan® Small RNA Assays Protocol (PN 4364031E).pdf, part nbr 4364031 Rev E 01/2011 and Taqman® MicroRNA assay RT primer [60X], catalog nbr 4440888 (large format). Incubations were performed in a Bio-Rad T100 thermo-cycler according to the manufacturer recommendations (Bio-Rad T100 thermal cycler Cat nbr 186-1096.pdf). cDNAs were stored in low binding tubes at -20°C until further use.

*- Quantitative PCR*

Expression of mature miRNAs was quantified according to the manufacturer's instructions using the Taqman miRNA RT-qPCR Assay 20X and TaqMan Universal Master Mix II, no Uracil-N-Glycosidase (UNG) 2X (Applied Biosystems, Life Technologies, Carlsbad, CA) in PCR Plate ThermoFisher 96-well, clear well, semi-skirted, catalog nbr AB-0900. A fixed volume of 5 μL total RNA was used as a template for the qPCR assay using a CFX96TM Real-Time System- C1000 - IVD certified according to manufacturer guidelines (Bio-Rad CFX96 Touch_Instruction manual.pdf, part nbr 110021337, Rev E US/EG). The hsa-miR-34a-5p TaqMan assay was used. The RT product from synthetic miRNAs was serially diluted and PCR was performed on all samples (standards and serum-derived RNA). The Cq Determination mode was Regression.

The sequences of mature miRNA and Taq Man assay ID are reported in the following table:

| miRNA ID | Sequence | miRbase Number | Assay ID |
|---|---|---|---|
| cel-miR-39-3p | UCACCGGGUGUAAAUCAGCUUG (SEQ ID NO: 1) | MIMAT0000010 | 000200 |
| hsa-miR-34a-5p | UGGCAGUGUCUUAGCUGGUUGU (SEQ ID NO:2) | MIMAT0000255 | 000426 |

## RESULTS

**A. The complexation of invivofectamine® with hsa-miR-34a-5p protects the synthetic miRNA from degradation.**

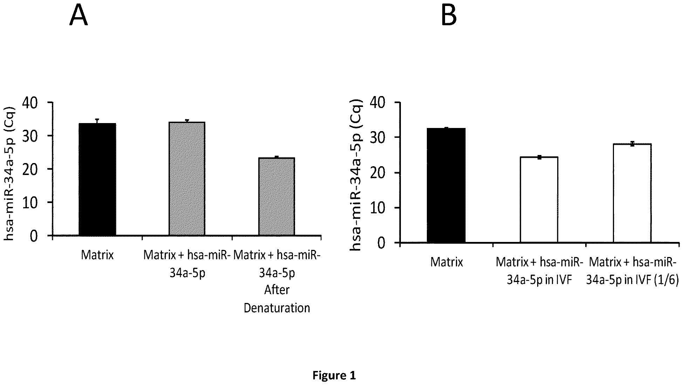

**Figure 1: invivofectamine® protects the synthetic hsa-miR-34a-5p oligoribonucleotide spiked in serum.**

hsa-miR-34a-5p expression levels in the absence (A) or the presence (B) of Invivofectamine®. Black box: pool of serum from healthy donors with very low levels of hsa-miR-34a used as a biological base matrix, Grey Box: base matrix spiked with synthetic hsa-miR-34a-5p without invivofectamine® (IVF) before or after denaturation. White box: base matrix spiked with synthetic hsa-miR-34a-5p encapsulated in IVF.

hsa-miR-34a-5p is detected in healthy patients (Matrix) at very low levels (33.6 Cq). The addition of a synthetic hsa-miR-34a-5p used as spike-in after addition of denaturating buffer in serum results in an induction (23.3 Cq). The synthetic miRNA is degraded if the addition occurs before the treatment of serum with the denaturating buffer (Matrix + hsa-miR-34a-5p). Interestingly, the combination of invivofectamine® with hsa-miR-34a-5p before the 1:6 dilution restores the miRNA level detected when the spike-in miRNA is added after denaturing buffer. The complex miRNA/invivofectamine® may be used with the 1/6 diluted complex (Figure 1).

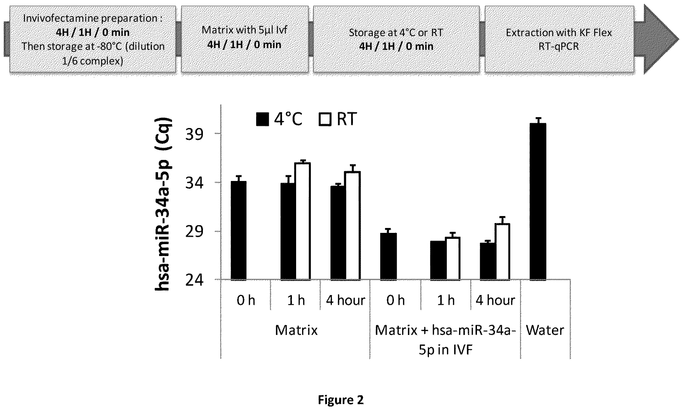

**Figure 2: miRNA/invivofectamine® complex stability at 4°C up to 4 hrs.**

Two miRNA/invivofectamine® complex preparation conditions were tested: temperature 4°C (black boxes) versus room temperature (RT, white boxes) and time between complex preparation and its use (0, 1 and 4 hours). Matrix: pool of serum from healthy donors (Etablissement Français du Sang: EFS) with very low levels of hsa-miR-34a-5p.

At room temperature, the hsa-miR-34a-5p levels decrease in matrix and in matrix with spike in hsa-miR-34a-5p. The levels remain constant at 4°C. This result suggests that spike-in procedure in blood derived samples may be delayed at 4°C after miRNA/invivofectamine® complex preparation (Figure 2).

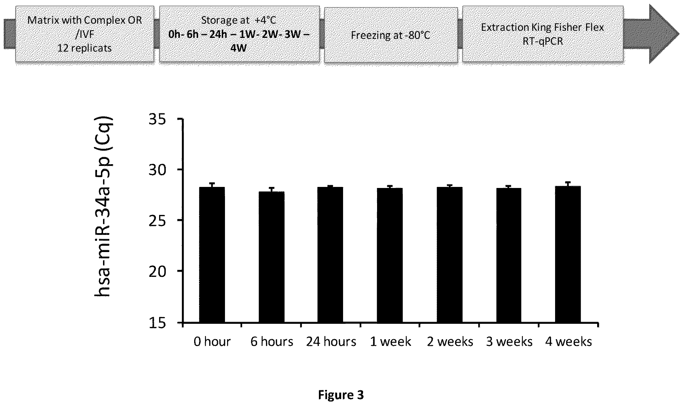

**Figure 3: Invivofectamine® protects the synthetic hsa-miR-34a-5p oligoribonucleotide spiked in serum at 4°C up to 4 weeks.**

The hsa-miR-34a-5p levels are stable in the spiked serum with the hsa-miR-34a-5p/invivofectamine® complex up to 4 weeks at 4°C (Figure 3).

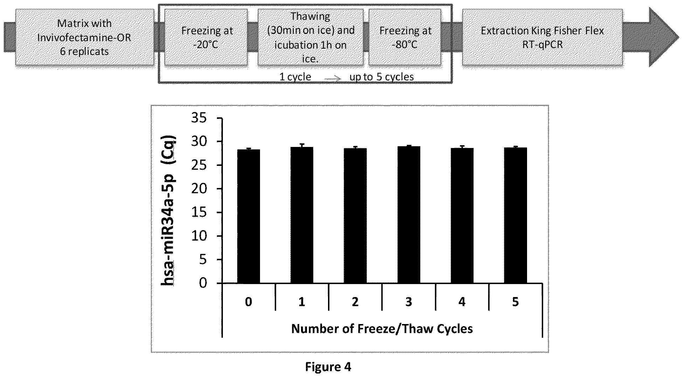

**Figure 4: Synthetic miRNA stability in serum when combined with invivofectamine® after repetitive freeze-thaw cycles at -20°C.**

5 freeze/thaw cycles were tested. Hsa-miR-34a-5p levels are not affected by repetitive freeze/thaw cycles at -20°C when the synthetic miRNA is combined with invivofectamine®.

**B. Robustness of the hsa-miR-34a-5p assay using miRs/IVF complexes**

**Table 1: Intra- and inter-assay variabilities of hsa-miR-34a-5p measured using the standard positive control (n=12 for each test condition) and cel-miR-39-3p/IVF complex as an internal control (spiked in each sample).** Samples were incubated at 4 °C for 4h or direct freezing at -80 °C.

| | Intra-Assay Variability | | | | | | | | Inter-Assay Variability | | | |
| | Experiment #1 | | | | Experiment #2 | | | | Experiment #1+2 | | | |
| | Cel-miR-39-3p | | hsa-miR-34a-5p | | Cel-miR-39-3p | | hsa-miR-34a-5p | | Cel-miR-39-3p | | hsa-miR-34a-5p | |
| | 4°C | -80°C | 4°C | -80°C | 4°C | -80°C | 4°C | -80°C | 4°C | -80°C | 4°C | -80°C |
|---|---|---|---|---|---|---|---|---|---|---|---|---|
| Mean (Cq) | 28.30 | 28.13 | 31.40 | 31.25 | 28.34 | 28.07 | 31.38 | 31.17 | 28.32 | 28.10 | 31.39 | 31.21 |
| SD (Cq) | 0.12 | 0.08 | 0.28 | 0.26 | 0.19 | 0.12 | 0.42 | 0.24 | 0.15 | 0.10 | 0.35 | 0.25 |
| CV (%) | 0.41 | 0.28 | 0.89 | 0.84 | 0.66 | 0.41 | 1.33 | 0.78 | 0.54 | 0.36 | 1.11 | 0.81 |
| Mean (fold) | 1.01 | | 1.12 | | 1.02 | | 1.17 | | 1.02 | | 1.15 | |
| SD (fold) | 0.16 | | 0.17 | | 0.23 | | 0.16 | | 0.2 | | 0.17 | |
| CV (%) | 16.01 | | 15.47 | | 22.37 | | 13.92 | | 19.31 | | 14.41 | |

Data are expressed as mean of Cq +/- SD or as fold hsa-miR-34a-p level expression using the following formula:

$$\text{hsa-miR-34a-p levels} = 2^{-\Delta\Delta Cq}$$

Where:

$$\Delta\Delta Cq = \Delta Cq_{sample} - \Delta Cq_{Standard}$$

$$\Delta Cq_{sample} = Cq_{hsa\text{-}miR\text{-}34a\text{-}5p \text{ of the sample}} - Cq_{cel\text{-}miR\text{-}39\text{-}3p \text{ spiked in the samples}}$$

$$\Delta Cq_{standard} = Cq_{hsa\text{-}miR\text{-}34a\text{-}5p \text{ of the positive standard}} - Cq_{cel\text{-}miR\text{-}39\text{-}3p \text{ spiked in the positive standard}}$$

Positive standards are pooled serum from NASH patients, n=12.

The spike-in miRNA is a non-human miRNA: cel-miR-39-3p at Cq=28. Invivofectamine® is used at ratio of cel-miR-39-3p/IVF = 100/100.

After preparation, the complex IVF/cel-miR-39 is incubated at 4 °C for 4h or directly freezed at -80 °C.

CV are not significantly different between conditions. The normalized quantification of hsa-miR-34a-5p remains stable and reproducible between conditions.

The internal control production is robust.

| | Internal Control = Cel-miR-39-3p in IVF | | |
| --- | --- | --- | --- |
| | hsa-miR-34a-5p (Cq) | Cel-miR-39-3p (Cq) | hsa-miR-34a-5p (fold=2$^{-\Delta\Delta Cq}$) |
| **Mean** | 31.43 | 28.24 | 1.01 |
| **Sd** | 0.61 | 0.54 | 0.15 |
| **CV** | 1.93% | 1.91% | **14.60%** |

**Table 2: Comparison of hsa-miR-34a-5p measurement variability** using the standard positive control (n=12 for each test condition) and cel-miR-39-3p/IVF or Cel-miR-40-3p/IVF complex as internal controls (spiked in each sample). Data are expressed as mean of Cq +/- SD or as fold hsa-miR-34a-5p level.

| | Internal Control = Cel-miR-40-3p in IVF | | |
| --- | --- | --- | --- |
| | hsa-miR-34a-5p (Cq) | Cel-miR-40-3p (Cq) | hsa-miR-34a-5p (fold=2$^{-\Delta\Delta Cq}$) |
| **Mean** | 31.37 | 28.23 | 1.01 |
| **Sd** | 0.84 | 0.81 | 0.11 |
| **CV** | 2.69% | 2.87% | **10.90 %** |

C. Performances of the hsa-miR-34a-5p assay.

Standard and internal controls were used to evaluate the performances of the hsa-miR-34a-5p assay in NASH patients.

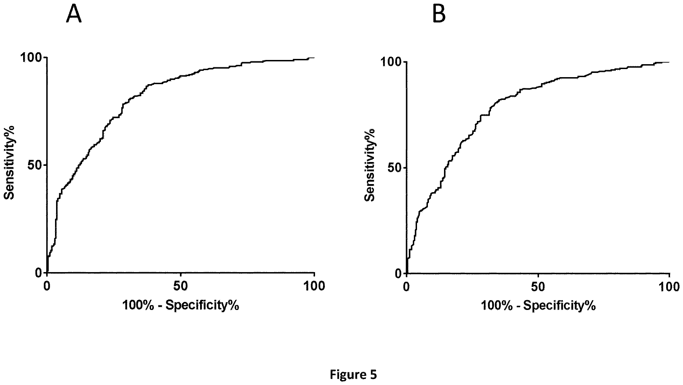

**Figure 5: ROC curve of hsa-miR-34a-5p with internal control (A) or without internal control (B) in clinical cohort with NASH diseased patients (n=562 patients, NTBT = 271 patients and TBT = 291 patients). Target condition to be classified as TBT was NAS≥4 + F≥2.**

The serum of 562 patients of the RESOLVE-IT study with corresponding liver biopsy was processed for the validation of the assay with an internal control. Serum samples from all patients were used to evaluate the performances of hsa miR-34a-5p assay using internal control (Cel-miR-40-3p) complexed to IVF and then compared of the test to the hsa-miR-34a-5p assay without the use of internal control. Patients were divided into 2 groups: group#1: Not To be Treated (NTBT) patients, with NAS score <4 and fibrosis <2 and group#2: To be Treated Patients (TBT), with NAS score ≥ 4 and fibrosis ≥2. Patients with NAS score 4 to 6 and fibrosis grade from 1 to 3 were the more representative of this clinical cohort. Patients with target condition (TBT: NAS≥4 + F≥2) represent 51.8% of total cohort population.

**Table3: Area under the curve characteristics to diagnose TBT patients with NAS≥4 and F≥2.**

| Condition | With Internal Control | Without Internal Control |
|---|---|---|
| Area | 0,8108 | 0,7838 |
| Std. Error | 0,01813 | 0,01935 |
| 95% confidence interval | 0,7753 to 0,8463 | 0,7458 to 0,8217 |
| P value | <0,0001 | <0,0001 |
| Data | | |
| Controls (NTBT2) | 271 | 271 |
| Patients (TBT2) | 291 | 291 |
| Missing Controls | 0 | 0 |
| Missing Patients | 0 | 0 |

The AUROC for hsa-miR-34a-5p assay using the internal control was 0.81 (95% CI 0.77-0.85) and the AUROC for hsa-miR-34a-5p assay where the internal control was not used represents only 0.78 (95% CI 0.74-0.82) indicating that hsa-miR-34a-5p assay using the internal control performs significantly better than the assay without internal control (Figure 5). Taken together all these data indicates that the hsa-miR-34a-5p assay is more specific and sensitive when Cel-miR-40-3p/IVF complex is used to quantify hsa-miR-34a-5p as a marker for the NASH disease.

## C) Comparison Invivofectamine®/Lipofectamine

*a-* Invivofectamine® or Lipofectamine/miRs complex preparation

Highly purified miRs mimic oligoribonucleotides are custom synthesized from IDT (Integrated DNA Technologies Skokie, Illinois USA). For *in vitro* testing as standards or internal controls, 100 µL of miRs mimic solution is prepared by mixing 50µ L miRs in RNase-free water (ref 733-2573, VWR) with 50 µL of complexation buffer (Invivofectamine® 3.0 reagent (IVF3001-3005, ThermoFisher Scientific). For example, use miR at 12.5 fmol/ml to prepare Control 28Cqs, miR at 2.5 fmol/ml to prepare Control 30Cqs and miR at 0.5 fmol/ml to prepare 33Cqs. Afterwards, diluted miRs are immediately added to 20 µL of invivofectamine® 3.0 or Lipofectamine (life Technologies Carlsbad, CA, USA) previously brought to room temperature. Invivofectamine® (or Lipofectamine) and diluted miRs are then vortexed for 3 seconds to ensure miRs-IVF 3.0 (or miRs-Lipofectamine) complex formation. Next, the invivofectamine® (or Lipofectamine)-miRs mixture is incubated for 30 min at 50°C and finally, the complex is diluted 6-fold by adding 1 mL PBS (pH 7.4) to obtain a final concentration respectively of 0,52 fmol/ml, 104 amol/and 20,8 amol/ml for Control 28Cqs, 30Cqs and 33Cqs. The preparation is conserved frozen at -20°C in aliquots.

To prepare standards miRs or internal controls, 5 µL of preparation are added to 1) biological base matrices for standard preparations (i.e. serum or plasma from healthy donors, or commercially available base matrices) or 2) directly to biological samples as internal control.

Comparison Invivofectamine®/Lipofectamine complex conditions

Several Invivofectamine®/hsa-miR-34-a-5p or Lipofectamine /hsa-miR-34-a-5p complex preparation conditions were tested: temperature ; (4°C versus room temperature) and time between complex preparation and its use (0, 1 and 4 hours). The matrix is a pool of serum from healthy donors with very low levels of hsa-miR-34a-5p. The standard RNA is a pool of RNA with a known hsa-miR-34a-5p levels. All Data are expressed as mean qPCR amplification cycles (Cq) +/- SD with n=3. ND: Not detectable.

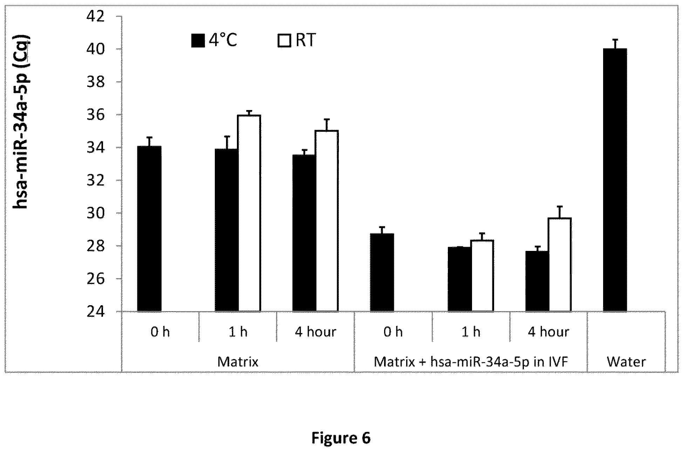

**Figure 6: Invivofectamine® (IVF)/hsa-miR-34-a-5p complex condition preparations.**

Temperature ; 4°C (black boxes), room temperature (RT, white boxes). Time between complex preparation and its use was also tested (0, 1 and 4 hours).

NRT: No Reverse Transcriptase control, NTC: No Template Control, Negative Control: PCR blank. All Data are expressed as mean qPCR amplification cycles (Cq) +/- SD with n=3. ND: Not detectable.

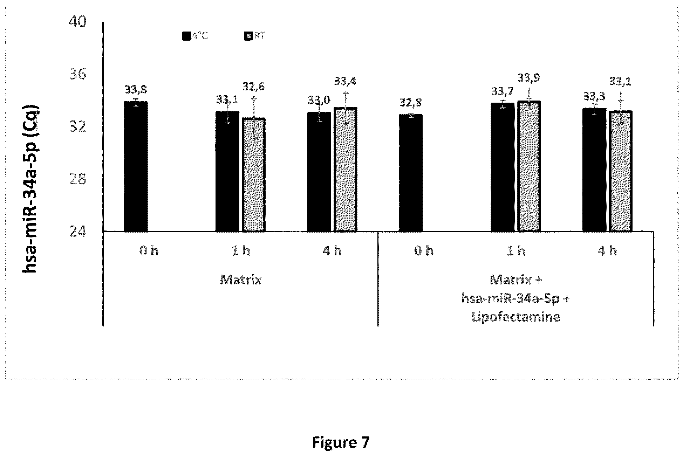

**Figure 7: Lipofectamine/hsa-miR-34-a-5p complex condition preparations.**

Temperature ; 4°C (black boxes), room temperature (RT, Grey boxes). Time between complex preparation and its use (0, 1 and 4 hours).

NRT: No Reverse Transcriptase control, NTC: No Template Control, Negative Control: PCR blank. All Data are expressed as mean qPCR amplification cycles (Cq) +/- SD with n=3. ND: Not detectable.

**Conclusion:** In all conditions (temperature and time), no protection of miR-34a-5p was observed with Lipofectamine. On the contrary, the use of Invivofectamine® protects the complex matrix + hsa-miR34a-5p.

**Claims**

1. A diagnostic kit comprising more than one container comprising a complex of a synthetic microRNA (miRNA) and a lipid vector, wherein said synthetic miRNA is at a defined concentration, wherein the lipid vector is selected from lipids conjugated to PEG,
   wherein the kit comprises:

      - one or more container(s) comprising a first synthetic miRNA; and
      - one or more container(s) comprising a second synthetic miRNA different from the first miRNA.

2. The diagnostic kit according to claim 1, wherein the kit comprises:

      - a first set of containers containing a first synthetic miRNA, wherein said first synthetic miRNA is at a different concentration in each container of the first set of containers; and
      - a second set of containers containing a second synthetic miRNA, wherein said second synthetic miRNA is at a different concentration in each container of the second set of containers.

3. The diagnostic kit according to claim 1 or 2, wherein at least one of the one or more container(s) comprises hsa-miR-34a-5p, hsa-miR-193b-3p, hsa-miR-452-5p, cel-miR39-3p or cel-miR-40-3p.

4. The diagnostic kit according to any one of claims 1 to 3, wherein the lipid vector further comprises a neutral lipid.

5. The diagnostic kit according to any one of claims 1 to 4 comprising more than one container comprising a complex of a synthetic microRNA (miRNA), a lipid vector, and a matrix selected in the group consisting of synthetic serum, plasma depleted in nucleic acid and blood derived sample, wherein said synthetic miRNA is at a defined concentration.

6. Use of the diagnostic kit according to any one of claims 1 to 5, in a method for the quantification of a miRNA in a biological fluid of a subject, in particular a mammal, more particularly a human.

7. Use according to claim 6, wherein quantification is relative or absolute quantification.

8. Use according to claim 6, wherein quantification is normalized relative or absolute quantification.

9. Use according to claims 7 or 8, wherein the kit is used to establish a standard concentration curve of the miRNA.

10. Use according to claim 6 or 8, wherein the kit is used to spike-in into the biological sample to be tested a defined amount of the synthetic miRNA comprised in a container of the kit.

11. The use according to any one of claims 6 to 10, wherein the biological sample is blood, serum, plasma, urine, saliva or sperm, in particular blood, serum or plasma, more particularly serum.

**Patentansprüche**

1. Diagnosekit umfassend mehr als einen Behälter, umfassend einen Komplex aus einer synthetischen MicroRNA (miRNA) und einem Lipidvektor, wobei die synthetische miRNA in einer definierten Konzentration vorliegt, wobei der Lipidvektor aus an PEG konjugierten Lipiden ausgewählt ist,
   wobei das Kit umfasst:

      - einen oder mehrere Behälter umfassend eine erste synthetische miRNA; und
      - einen oder mehrere Behälter umfassend eine zweite synthetische miRNA, die sich von der ersten miRNA unterscheidet.

2. Das Diagnosekit nach Anspruch 1, wobei das Kit umfasst:

- einen ersten Behältersatz, der eine erste synthetische miRNA enthält, wobei diese erste synthetische miRNA in jedem Behälter des ersten Behältersatzes in einer anderen Konzentration vorliegt; und
- einen zweiten Behältersatz, der eine zweite synthetische miRNA enthält, wobei diese zweite synthetische miRNA in jedem Behälter des zweiten Behältersatzes in einer anderen Konzentration vorliegt.

3. Das Diagnosekit nach Anspruch 1 oder 2, wobei mindestens einer der einen oder mehreren Behälter hsa-miR-34a-5p, hsa-miR-193b-3p, hsa-miR-452-Sp, cel-miR39-3p oder cel-miR-40-3p umfasst.

4. Das Diagnosekit nach einem der Ansprüche 1 bis 3, wobei der Lipidvektor außerdem ein neutrales Lipid umfasst

5. Das Diagnosekit nach einem der Ansprüche 1 bis 4, umfassend mehr als einen Behälter umfassend einen Komplex aus einer synthetischen microRNA (miRNA), einem Lipidvektor und einer Matrix, die aus der Gruppe bestehend aus synthetischem Serum und an Nukleinsäuren abgereichertem Plasma und von Blut stammenden Proben ausgewählt ist, wobei diese synthetische miRNA in einer definierten Konzentration vorliegt.

6. Verwendung des Diagnosekits nach einem der Ansprüche 1 bis 5 in einem Verfahren zur Quantifizierung einer miRNA in einer biologischen Flüssigkeit eines Subjekts, insbesondere eines Säugetiers, ganz insbesondere eines Menschen.

7. Verwendung nach Anspruch 6, wobei die Quantifizierung eine relative oder absolute Quantifizierung ist.

8. Verwendung nach Anspruch 6, wobei die Quantifizierung normalisiert-relative oder absolute Quantifizierung ist.

9. Verwendung nach Anspruch 7 oder 8, wobei das Kit zur Erstellung einer Standardkonzentrationskurve der miRNA verwendet wird.

10. Verwendung nach Anspruch 6 oder 8, wobei das Kit zum Einbringen einer definierten Menge der in einem Behälter des Kits enthaltenen synthetischen miRNA in die zu testende biologische Probe verwendet wird.

11. Verwendung nach einem der Ansprüche 6 bis 10, wobei die biologische Probe Blut, Serum, Plasma, Urin, Speichel oder Sperma, insbesondere Blut, Serum oder Plasma, insbesondere Serum, ist

## Revendications

1. Trousse de diagnostic comprenant plusieurs récipients comprenant un complexe d'un microARN (miARN) synthétique et d'un vecteur lipidique, dans laquelle ledit miARN synthétique est à une concentration définie, dans laquelle le vecteur lipidique est choisi parmi les lipides conjugués à du PEG,
laquelle trousse comprend :

- un ou plusieurs récipients comprenant un premier miARN synthétique ; et
- un ou plusieurs récipients comprenant un deuxième miARN synthétique différent du premier miARN.

2. Trousse de diagnostic selon la revendication 1, laquelle trousse comprend :

- un premier jeu de récipients contenant un premier miARN synthétique, dans lequel ledit premier miARN synthétique est à une concentration différente dans chaque récipient du premier jeu de récipients ; et
- un deuxième jeu de récipient contenant un deuxième miARN synthétique, dans lequel ledit deuxième miARN synthétique est à une concentration différente dans chaque récipient du deuxième jeu de récipients.

3. Trousse de diagnostic selon la revendication 1 ou 2, dans laquelle au moins l'un parmi le ou les récipients comprend hsa-miR-34a-5p, hsa-miR-193b-3p, hsa-miR-452-Sp, cel-miR39-3p ou cel-miR-40-3p.

4. Trousse de diagnostic selon l'une quelconque des revendications 1 à 3, dans laquelle le vecteur lipidique comprend en outre un lipide neutre.

5. Trousse de diagnostic selon l'une quelconque des revendications 1 à 4, comprenant plusieurs récipients comprenant un complexe d'un microARN (miARN) synthétique, d'un vecteur lipidique, et d'une matrice choisie dans le groupe constitué par un sérum synthétique, un plasma appauvri en acide nucléique et un échantillon dérivé de sang, dans laquelle ledit miARN synthétique est à une concentration définie.

6. Utilisation de la trousse de diagnostic de l'une quelconque des revendications 1 à 5, dans une méthode pour la quantification d'un miARN dans un fluide biologique d'un sujet, en particulier d'un mammifère, plus particulièrement d'un humain.

7. Utilisation selon la revendication 6, dans laquelle la quantification est une quantification relative ou absolue.

8. Utilisation selon la revendication 6, dans laquelle la quantification est une quantification relative ou absolue normalisée.

9. Utilisation selon la revendication 7 ou 8, dans laquelle la trousse est utilisée pour établir une courbe de concentration standard du miARN.

10. Utilisation selon la revendication 6 ou 8, dans laquelle la trousse est utilisée pour insérer dans l'échantillon biologique devant être testé une quantité définie du miARN synthétique compris dans un récipient de la trousse.

11. Utilisation selon l'une quelconque des revendications 6 à 10, dans laquelle l'échantillon biologique est le sang, le sérum, le plasma, l'urine, la salive ou le sperme, en particulier, le sang, le sérum ou le plasma, plus particulièrement le sérum.

Figure 1

Figure 2

| Matrix with Complex OR /IVF 12 replicats | Storage at +4°C **0h- 6h – 24h – 1W- 2W- 3W – 4W** | Freezing at -80°C | Extraction King Fisher Flex RT-qPCR |

**Figure 3**

Figure 4

A

B

Figure 5

**Figure 6**

**Figure 7**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013148151 A
- WO 9405624 A
- WO 9427435 A
- WO 9502698 A
- WO 9517373 A
- WO 9640961 A
- US 8058068 B
- WO 9840502 A
- US 20060229246 A
- US 20030069173 A

- WO 200027795 A
- WO 200234879 A
- WO 2006102163 A
- WO 201268176 A
- WO 201611203 A
- US 20190060482 A
- WO 2012068176 A
- WO 2016011203 A
- WO 2017167934 A

**Non-patent literature cited in the description**

- **GUO ZE et al.** *Journal of Dermatological Science,* 2013, vol. 69, 68-76